# DEMANDE DE BREVET EUROPEEN

(11) **EP 3 620 507 A1**
(43) Date de publication de la demande: **11.03.2020**
(21) Numéro de dépôt: 19195910.5
(22) Date de dépôt: 06.09.2019
(51) Int. Cl.: C12M 1/107, C05C 11/00, C12M 1/00, C12P 3/00

(54) **PROCEDE BIOLOGIQUE DE PRODUCTION D'AZOTE MINERAL ET/OU DE DIHYDROGENE A PARTIR DE DIAZOTE ATMOSPHERIQUE**

(30) Priorité: 06.09.2018 FR 1858005
(71) Demandeur: MEC, 54610 Belleau (FR)
(72) Inventeur: NOIROT, Fabrice, 54610 BELLEAU (FR); MILLARDET, Franck Christophe Stéphane, 54670 CUSTINES (FR); PAQUIN, Laurent, 54150 NORROY LE SEC (FR); PAQUIN, Eric, 54190 FLEVILLE LIXIERES (FR)
(74) Mandataire: Lavoix

(57) **Abrégé**

La présente invention a pour objet un procédé biologique de production d'azote minéral et/ou de dihydrogène à partir du diazote atmosphérique, en utilisant successivement des bactéries fixatrices d'azote et des bactéries du genre *Nitrosomonas* et/ou du genre *Nitrobacter.* La présente invention a également pour objet un dispositif approprié pour la mise en oeuvre de ce procédé et comprenant au moins trois compartiments successifs. La présente invention a particulièrement pour objet un dispositif comprenant un bioréacteur (1), ledit bioréacteur comprenant une cuve de fermentation (24) comprenant au moins trois compartiments successifs (3, 4, 5). La présente invention a également pour objet l'utilisation de bactéries fixatrices d'azote et/ou de bactéries du genre *Nitrosomonas* et/ou du genre *Nitrobacter* pour la production d'azote minéral et/ou de dihydrogène.

## Description

### Domaine technique

La présente invention concerne la production d'azote minéral assimilable par des plantes qui ne sont pas des légumineuses.

### Etat de la technique

Pour sa croissance, une plante a besoin d'eau, de dioxyde de carbone et de nutriments, tels qu'une source d'azote, phosphore, potassium, soufre, magnésium, calcium et d'oligo-éléments. Une carence en eau et/ou en un ou plusieurs de ces nutriments va impacter négativement la croissance d'une plante.

Ces nutriments sont généralement puisés dans le sol par les racines de la plante, en même temps que l'eau. Ils peuvent également être apportés sous la forme de produits fertilisants, afin d'optimiser la croissance des plantes. En agriculture, cela permet notamment d'augmenter le rendement et la qualité des cultures.

L'azote constitue, avec le phosphore et le potassium, un élément constitutif majeur d'une plante. L'azote est présent dans l'air sous la forme de diazote (N2) ou dans le sol sous forme minérale (par exemple ammoniaque NH₄OH, nitrate NO₃⁻) ou organique (par exemple humus). L'azote organique n'est pas directement assimilable par les plantes. Les légumineuses ont la propriété de pouvoir utiliser directement l'azote de l'air, grâce aux bactéries présentes dans leurs racines au niveau des nodules. Les autres plantes sont incapables d'utiliser l'azote contenu dans l'air et utilisent uniquement l'azote minéral du sol.

Les engrais azotés contiennent généralement de l'ammoniac, du nitrate d'ammonium ou de l'urée.

L'ammoniac (NH3) est par exemple produit par la réaction de l'azote de l'air avec de l'hydrogène provenant du gaz naturel, à haute pression et température (par exemple entre 200 et 400 bars et à environ 450 °C).

Le nitrate d'ammonium et l'urée sont obtenus à partir de l'ammoniac.

Par exemple, le nitrate d'ammonium (NH₄NO₃) est obtenu par mélange d'ammoniac et de dioxygène pour obtenir de l'oxyde nitrique gazeux, absorption de l'oxyde nitrique gazeux dans l'eau pour obtenir de l'acide nitrique, puis mélange de l'acide nitrique et d'ammoniac gazeux à une température comprise de 100°C à 180°C, pour obtenir du nitrate d'ammonium.

L'urée (CO(NH2)2) est par exemple obtenu par réaction de l'ammoniac avec du dioxyde de carbone à haute pression.

Les procédés de production des engrais azotés actuellement utilisés sont ainsi des procédés chimiques qui consomment beaucoup d'énergie et nécessitent des dispositifs permettant de travailler à haute température et haute pression, notamment pour obtenir l'ammoniac. L'achat de ces engrais azotés obtenus par ces procédés représente ainsi un coût très important pour l'agriculteur.

Il existe donc un besoin de fournir des procédés alternatifs pour la production d'azote minéral directement assimilable par les plantes qui ne sont pas des légumineuses, ces procédés alternatifs étant de préférence moins consommateurs d'énergie, plus faciles de mise en oeuvre et/ou permettant de diminuer le coût pour les agriculteurs.

### Description détaillée

De manière surprenante, les Inventeurs ont mis en évidence qu'il est possible d'obtenir de l'azote minéral assimilable par des plantes qui ne sont pas des légumineuses, à partir de l'azote contenu dans l'air (i.e. de diazote N2) au moyen d'un procédé biologique original.

Le procédé selon l'invention présente de nombreux avantages. Il est notamment simple de mise en oeuvre et peut être utilisé par l'agriculteur afin de produire lui-même l'engrais azoté sur son exploitation, à moindre coût.

De manière avantageuse, le procédé selon l'invention permet également de produire de l'hydrogène, qui peut par exemple être utilisé dans le domaine de l'énergie, par exemple comme carburant.

Le procédé selon l'invention diffère notamment des procédés actuellement utilisés en ce qu'il s'agit d'un procédé biologique mettant en oeuvre des microorganismes qui utilisent directement le carbone de la matière organique comme énergie.

Le procédé selon l'invention peut être mis en oeuvre dans dispositif approprié comprenant un ou plusieurs bioréacteurs, comme décrit ci-après.

La présente invention a ainsi pour objet un procédé biologique de production d'azote minéral et/ou de dihydrogène, à partir de diazote, caractérisé en ce qu'il comprend les étapes suivantes :
a) mise en incubation de matière organique, de diazote (N2), d'une source de sucre simple et de bactéries fixatrices d'azote dans un bioréacteur, pour obtenir un milieu de fermentation comprenant de l'ammoniac (NH3),
b) mise en incubation du milieu de fermentation obtenu à l'étape a) en présence d'eau et avec retrait de dihydrogène (H2) du bioréacteur, pour obtenir un milieu de fermentation comprenant de l'ammonium (NH₄⁺),
c) mise en incubation du milieu de fermentation obtenu à l'étape b) en présence de dioxygène (O₂) et de bactéries du genre *Nitrosomonas* et/ou du genre *Nitrobacter* dans le bioréacteur, pour obtenir un milieu de fermentation comprenant des nitrates (NO₃⁻),
d) optionnellement, séparation du milieu de fermentation obtenu à l'étape c) en une phase liquide et une phase solide et élimination de la phase solide, pour obtenir une solution riche en nitrates,
e) optionnellement, concentration de la solution riche en nitrates obtenue à l'étape d), pour obtenir une solution concentrée en nitrates.

Les bactéries fixatrices d'azote sont de préférence sélectionnées dans le groupe consistant en des bactéries du genre *Azotobacter, Nitrobacter, Azospirillum, Acetobacter, Diazotrophicus, Clostridium, Klebsiella, Bacillus, Pseudomonas, Rhodobacter, Rhodospirillum, Synechoccus* et leurs combinaisons.

L'étape a) est de préférence effectuée en conditions anaérobies, à une température comprise de 30°C à 36°C, et/ou à une pression comprise de 0,1 ntm à 5 ntm.

Le procédé tel que défini ci-dessus est de préférence mis en oeuvre dans un dispositif tel que défini ci-après.

Un autre objet de l'invention concerne un dispositif approprié pour la mise en oeuvre d'un procédé biologique de production d'azote minéral et/ou de dihydrogène, à partir de diazote, caractérisé en ce que :
- le dispositif comprend au moins trois compartiments successifs, le premier compartiment 3, 3', 3" étant relié à une entrée 2 et le dernier compartiment 5, 5', 5" étant relié à une sortie 6, lesdits compartiments successifs étant reliés entre eux de sorte qu'un liquide introduit par l'entrée 2 passera successivement dans le premier compartiment 3, 3', 3", un ou des éventuel(s) autre(s) compartiment(s), le compartiment intermédiaire 4, 4', 4", un ou des éventuel(s) autre(s) compartiment(s) et le dernier compartiment 5, 5', 5" jusqu'à la sortie 6,
- le premier compartiment 3, 3', 3" comprend en outre une entrée 8 appropriée pour introduire un gaz, une sortie 9 appropriée pour évacuer un gaz et deux entrées 10, 27 appropriées pour introduire un liquide,
- le compartiment intermédiaire 4, 4', 4" comprend une sortie 11 appropriée pour évacuer un gaz et une entrée 12 appropriée pour introduire un liquide, et
- le dernier compartiment 5, 5', 5" comprend une entrée 13 appropriée pour réguler le pH, une entrée 14 appropriée pour introduire un gaz et une entrée 15 appropriée pour introduire un liquide.

L'ensemble des compartiments successifs du dispositif tel que défini ci-dessus peut par exemple constituer une cuve de fermentation 24. Alternativement, chacun des compartiments successifs du dispositif tel que défini ci-dessus peut constituer une cuve de fermentation ou un bioréacteur.

Le dispositif tel que défini ci-dessus peut avantageusement comprendre en outre un dispositif 18 permettant de séparer une phase liquide d'une phase solide et/ou un dispositif 21 permettant de concentrer une solution.

Dans un mode de réalisation préféré, le dispositif tel que défini ci-dessus comprend un bioréacteur 1, ledit bioréacteur comprenant une cuve de fermentation 24 comprenant une entrée 2 appropriée pour introduire un liquide et une sortie 6 appropriée pour évacuer un liquide et ladite cuve de fermentation comprenant au moins trois compartiments successifs, le premier compartiment 3 étant relié à l'entrée 2 et le dernier compartiment 5 étant relié à la sortie 6, lesdits compartiments successifs étant reliés entre eux de sorte qu'un liquide introduit par l'entrée 2 passera successivement dans le premier compartiment 3, un ou des éventuel(s) autre(s) compartiment(s), le compartiment intermédiaire 4, un ou des éventuel(s) autre(s) compartiment(s) et le dernier compartiment 5 jusqu'à la sortie 6.

Un autre objet de l'invention concerne l'utilisation d'un dispositif tel que défini ci-dessus pour la production d'azote minéral et/ou de dihydrogène à partir de diazote.

Un autre objet de l'invention concerne l'utilisation de bactéries fixatrices d'azote et/ou de bactéries du genre *Nitrosomonas* et/ou du genre *Nitrobacter* pour la production d'azote minéral et/ou de dihydrogène à partir de diazote.

### Dispositif

Le dispositif selon l'invention est un dispositif approprié pour la mise en oeuvre d'un procédé biologique de production d'azote minéral, en particulier de nitrates, à partie de diazote. Le dispositif selon l'invention comprend un bioréacteur ou plusieurs bioréacteurs.

Un bioréacteur, appelé également fermenteur, utilisé dans le cadre de l'invention, est un appareil permettant de multiplier des bactéries pour la production de métabolites d'intérêt.

Un bioréacteur selon l'invention peut comprendre une ou plusieurs cuves de fermentation.

La cuve de fermentation est une cuve adaptée à la fermentation de bactéries.

Toute cuve de fermentation appropriée bien connue de l'homme de métier peut être utilisée.

La cuve de fermentation est par exemple en acier inoxydable.

Le volume de la cuve de fermentation est par exemple compris de 1 m³ à 1000 m³, de préférence de 5 m³à 100 m³, plus préférentiellement de 10 m³ à 50 m³.

Le dispositif selon l'invention comprend au moins trois compartiments successifs : un premier compartiment (par exemple 3, 3' ou 3"), un compartiment intermédiaire (par exemple 4, 4' ou 4") et un dernier compartiment (par exemple 5, 5', 5").

Le premier compartiment est relié à une entrée 2 et le dernier compartiment est relié à une sortie 6, ladite entrée 2 et ladite sortie 6 étant telles qu'un liquide introduit dans le premier compartiment par l'entrée 2 ne peut pas ressortir par cette entrée et qu'un liquide sortant par la sortie 6 ne peut pas retourner dans le dernier compartiment. A cet effet, ladite entrée 2 et ladite sortie 6 sont de préférence munies chacune d'un clapet anti-retour 7.

Chaque compartiment est relié au compartiment suivant de manière à ce qu'un liquide introduit dans le compartiment suivant ne peut pas retourner dans le compartiment précédent. Par exemple, chaque compartiment est relié au compartiment suivant par au moins un clapet anti-retour 7.

Ainsi, un liquide introduit par l'entrée 2 passera successivement dans le premier compartiment, un ou des éventuel(s) autre(s) compartiment(s), le compartiment intermédiaire, un ou des éventuel(s) autre(s) compartiment(s) et le dernier compartiment jusqu'à la sortie 6. A cet effet, le dispositif peut comprendre un clapet anti-retour 7 entre les différents compartiments successifs, en particulier entre le premier compartiment et le compartiment intermédiaire et/ou entre le compartiment intermédiaire et le dernier compartiment.

Les compartiments successifs peuvent être de mêmes dimensions et/ou de même volume et/ou de même forme. Alternativement, deux, trois ou au moins trois compartiments peuvent avoir des dimensions différentes et/ou un volume différent et/ou une forme différente.

Un compartiment comprend de préférence une partie supérieure, une partie inférieure, deux parties opposées et deux parties latérales. Les compartiments successifs sont reliés entre eux au niveau de leurs parties opposées. De préférence, l'entrée 2 est située au niveau de la partie opposée libre du premier compartiment, c'est-à-dire de la partie opposée qui n'est pas reliée au compartiment suivant et/ou la sortie 6 est située au niveau de la partie opposée libre du dernier compartiment, c'est-à-dire de la partie opposée qui n'est pas reliée au compartiment précédent.

Dans un mode de réalisation préféré, le premier compartiment 3, 3', 3" comprend en outre une entrée 8, notamment appropriée pour introduire un gaz, de préférence sous pression, au moins une sortie 9, notamment appropriée pour évacuer un gaz et deux entrées 10 et 27, notamment appropriées pour introduire un liquide.

L'entrée 8 appropriée pour introduire un gaz et/ou la sortie 9 appropriée pour évacuer un gaz sont, de préférence, situées au niveau de la partie supérieure du premier compartiment.

Les entrées 10 et/ou 27 appropriées pour introduire un liquide sont situées, de préférence, en dessous du niveau d'introduction de l'entrée 2.

L'entrée 10 et/ou l'entrée 27 appropriées pour introduire un liquide peuvent être situées au niveau de la partie opposée libre, de la partie inférieure ou d'une partie latérale du premier compartiment, de préférence au niveau de la partie opposée libre du premier compartiment.

Dans un mode de réalisation préféré, le compartiment intermédiaire 4, 4', 4" comprend également une sortie 11, notamment appropriée pour évacuer un gaz et une entrée 12, notamment appropriée pour introduire un liquide.

La sortie 11 appropriée pour évacuer un gaz est, de préférence, située au niveau de la partie supérieure du compartiment intermédiaire.

L'entrée 12 appropriée pour introduire un liquide peut être située au niveau de la partie supérieure, inférieure ou d'une partie latérale du compartiment intermédiaire, de préférence au niveau de la partie supérieure.

Dans un mode de réalisation préféré, le dernier compartiment 5, 5', 5" comprend également une entrée 13 appropriée pour réguler le pH (par exemple permettant d'introduire un liquide), une entrée 14, notamment appropriée pour introduire un gaz et au moins une entrée 15, notamment appropriée pour introduire un liquide.

L'entrée 14 appropriée pour introduire un gaz est, de préférence, située au niveau de la partie supérieure du dernier compartiment.

L'entrée 13 appropriée pour réguler le pH et/ou l'entrée 15 appropriée pour introduire un liquide peuvent être situées au niveau de la partie supérieure, inférieure ou d'une partie latérale du compartiment intermédiaire. Par exemple, l'entrée 13 appropriée pour réguler le pH est située au niveau de la partie supérieure et/ou l'entrée 15 appropriée pour introduire un liquide est située au niveau de la partie inférieure du compartiment intermédiaire.

Le dispositif comprend, de préférence, un vérin ou un piston à spirale 16 relié à l'entrée 2, permettant ainsi d'injecter dans l'entrée 2 un liquide, en particulier de la matière organique, arrivant par l'entrée 17.

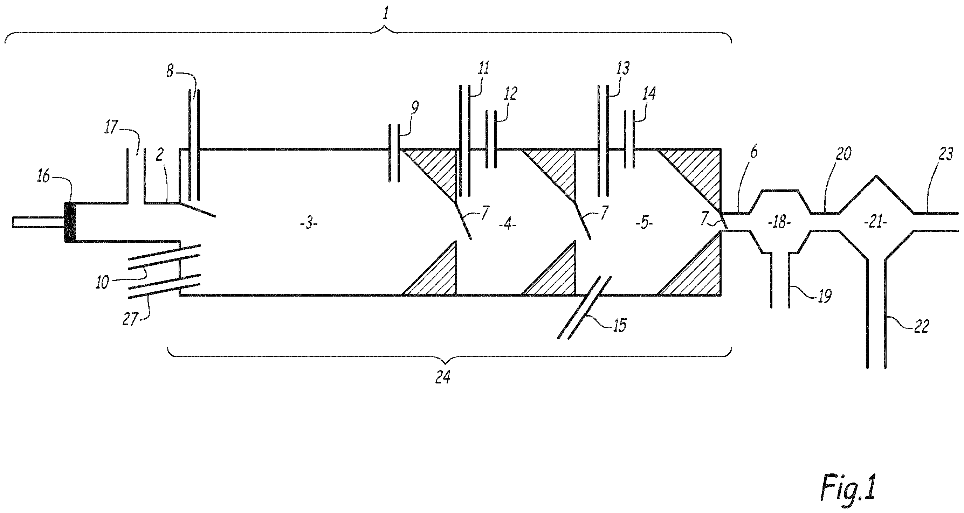

Dans un mode de réalisation préféré de l'invention, l'ensemble des compartiments successifs du dispositif constitue une cuve de fermentation 24. Dans ce cas, le dispositif comprend un bioréacteur 1, ledit bioréacteur comprenant une cuve de fermentation 24 et ladite cuve de fermentation comprenant ou consistant en au moins lesdits trois compartiments successifs (par exemple 3, 4 et 5) définis ci-dessus. Un exemple de ce mode de réalisation est illustré à la Figure 1.

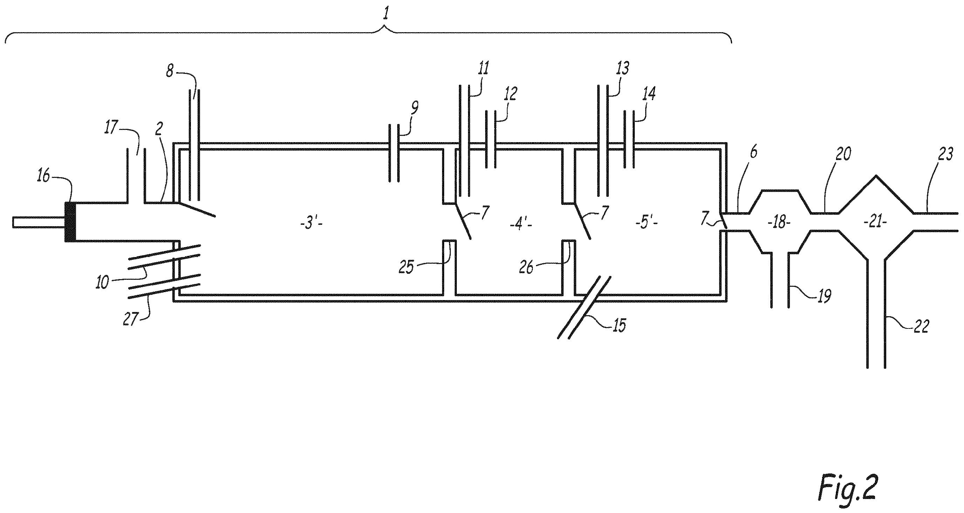

Dans un autre mode de réalisation préféré de l'invention, chacun des compartiments successifs constitue une cuve de fermentation distincte. Dans ce cas, le dispositif comprend un bioréacteur 1, ledit bioréacteur comprenant au moins trois cuves de fermentation montées en série, chaque cuve de fermentation correspondant à un compartiment (par exemple 3', 4' et 5') tel que défini ci-dessus. Un exemple de ce mode de réalisation est illustré à la Figure 2.

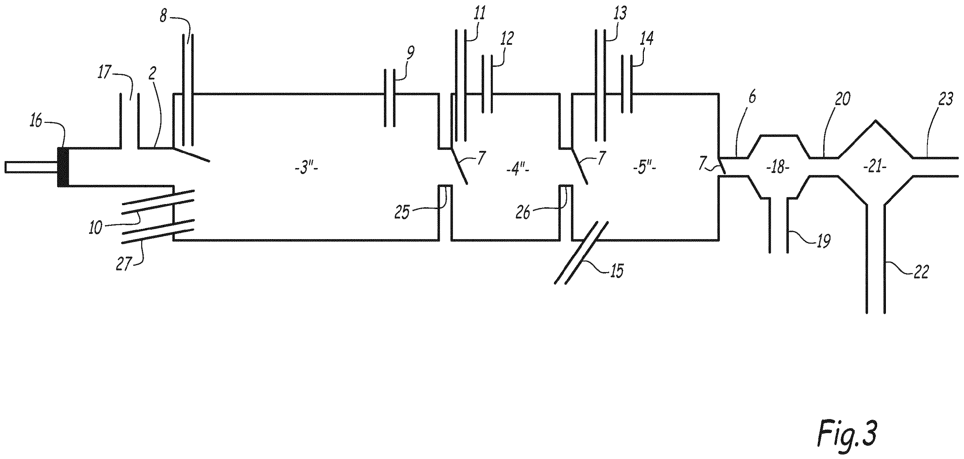

Dans un autre mode de réalisation préféré de l'invention, chacun des compartiments successifs constitue un bioréacteur distinct. Dans ce cas, le dispositif comprend au moins trois bioréacteurs successifs montés en série, chaque bioréacteur correspondant à un compartiment (par exemple 3", 4" et 5") tel que défini ci-dessus. Un exemple de ce mode de réalisation est illustré à la Figure 3.

Lorsque chacun des compartiments successifs constitue un bioréacteur distinct ou une cuve de fermentation distincte, lesdits compartiments successifs sont de préférence reliés entre eux par une conduite appropriée pour un liquide. Le premier compartiment et le compartiment intermédiaire sont par exemple reliés entre eux par une sortie 25, ladite sortie 25 étant de préférence munie d'au moins un clapet anti-retour 7 qui peut être placé à la sortie du premier compartiment, à l'entrée du compartiment intermédiaire ou dans la sortie 25, c'est-à-dire entre la sortie du premier compartiment et l'entrée du compartiment intermédiaire. Le compartiment intermédiaire et le dernier compartiment sont par exemple reliés entre eux par une sortie 26, ladite sortie 26 étant de préférence munie d'au moins un clapet anti-retour 7 qui peut être placé à la sortie du compartiment intermédiaire, à l'entrée du dernier compartiment ou dans la sortie 26, c'est-à-dire entre la sortie du compartiment intermédiaire et l'entrée du dernier compartiment.

La présente invention a ainsi particulièrement pour objet un dispositif tel que défini ci-dessus, caractérisé en ce que :
- ledit dispositif comprend au moins trois compartiments successifs, le premier compartiment 3, 3', 3" étant relié à une entrée 2 et le dernier compartiment 5, 5', 5" étant relié à une sortie 6, lesdits compartiments successifs étant reliés entre eux de sorte qu'un liquide introduit par l'entrée 2 passera successivement dans le premier compartiment 3, 3', 3", un ou des éventuel(s) autre(s) compartiment(s), le compartiment intermédiaire 4, 4', 4", un ou des éventuel(s) autre(s) compartiment(s) et le dernier compartiment 5, 5', 5" jusqu'à la sortie 6,
- le premier compartiment 3, 3', 3" comprend en outre une entrée 8 appropriée pour introduire un gaz, une sortie 9 appropriée pour évacuer un gaz et deux entrées 10, 27 appropriées pour introduire un liquide,
- le compartiment intermédiaire 4, 4', 4" comprend une sortie 11 appropriée pour évacuer un gaz et une entrée 12 appropriée pour introduire un liquide, et
- le dernier compartiment 5, 5', 5" comprend une entrée 13 appropriée pour réguler le pH, une entrée 14 appropriée pour introduire un gaz et une entrée 15 appropriée pour introduire un liquide.

Dans un mode de réalisation avantageux, chacun des compartiments successifs comprend au moins un des capteurs ou système suivants:
- un capteur de température et/ou d'un régulateur de température, en particulier pour le premier compartiment (par exemple 3, 3', 3"), le compartiment intermédiaire (par exemple 4, 4', 4") et/ou le dernier compartiment (par exemple 5, 5', 5"),
- un capteur de pH et/ou des moyens pour réguler le pH (par exemple une entrée pour une base et/ou une entrée pour un acide), en particulier pour le dernier compartiment 5, 5', 5",
- un capteur de pression et/ou un régulateur de pression, en particulier pour le premier compartiment 3, 3', 3",
- un capteur de niveau de liquide et/ou un régulateur du niveau de liquide,
- un capteur de mesure de la concentration en hydrogène (par exemple un capteur ampérométrique H2),
- un capteur de mesure de la concentration en oxygène dissous (par exemple sonde oxymétrique) et/ou
- un système d'agitation, par exemple comprenant une ou plusieurs turbines.

Lorsque la cuve comprend plus de trois compartiments, le ou les compartiments additionnels, - c'est-à-dire autre que le premier compartiment, le compartiment intermédiaire et le dernier compartiment -, est un doublon du premier compartiment, du compartiment intermédiaire et/ou du dernier compartiment. Dans un tel mode de réalisation, les compartiments qui sont des doublons sont placés l'un à la suite de l'autre. Ainsi, un liquide introduit par l'entrée 2, passera successivement dans le premier compartiment, éventuellement par au moins un doublon du premier compartiment, puis par le compartiment intermédiaire, puis éventuellement au moins un doublon du compartiment intermédiaire, puis éventuellement au moins un doublon du dernier compartiment, puis par le dernier compartiment 5.

Le doublon du compartiment intermédiaire est de préférence identique au compartiment intermédiaire.

Le doublon du premier compartiment diffère de préférence du premier compartiment en ce qu'il comprend au total deux entrées, en particulier 10 et 27 appropriées pour l'introduction d'un liquide (au lieu de trois entrées). De préférence, le doublon du premier compartiment ne comprend pas une entrée 2 appropriée pour l'introduction d'un liquide, notamment de la matière organique.

Le doublon du dernier compartiment diffère de préférence du dernier compartiment en ce qu'il ne comprend pas de sortie 6 appropriée pour un liquide.

Dans un mode de réalisation avantageux de l'invention, le dispositif comprend en outre :
- un dispositif 18 permettant de séparer une phase liquide d'une phase solide et/ou
- un dispositif 21 permettant de concentrer une solution.

Le dispositif 18 est de préférence relié au dernier compartiment par la sortie 6.

Le dispositif 18 est de préférence une centrifugeuse ou un séparateur de phase.

Le dispositif 18 comprend de préférence une sortie 20 appropriée pour évacuer une phase liquide et une sortie 19 appropriée pour évacuer une phase solide.

Le dispositif 21 permettant de concentrer une solution est de préférence relié au dispositif 18 permettant de séparer une phase liquide d'une phase solide par la sortie 20.

Le dispositif 21 permettant de concentrer une solution comprend par exemple une ou plusieurs membranes appropriées pour concentrer une solution par filtration, de préférence ultrafiltration, ou osmose inverse.

Le dispositif 21 comprend de préférence deux sorties 22 et 23.

La sortie 22 permet de récupérer le rétentat, c'est-à-dire une solution concentrée en nitrates et autres formes azotées, et la sortie 23 d'évacuer le perméat.

La présente invention a ainsi particulièrement pour objet un dispositif tel que défini ci-dessus, caractérisé en ce qu'il comprend ou consiste en a) un bioréacteur 1, b) optionnellement, un dispositif 18 permettant de séparer une phase liquide d'une phase solide et c) optionnellement, un dispositif 21 permettant de concentrer une solution, et en ce que :
- ledit bioréacteur comprend une cuve de fermentation 24 comprenant une entrée 2 appropriée pour introduire un liquide et une sortie 6 appropriée pour évacuer un liquide,
- ladite cuve de fermentation comprend au moins trois compartiments successifs 3, 4 et 5, le premier compartiment 3 étant relié à l'entrée 2 et le dernier compartiment 5 étant relié à la sortie 6, lesdits compartiments successifs étant reliés entre eux de sorte qu'un liquide introduit par l'entrée 2 passera successivement dans le premier compartiment 3, un ou des éventuel(s) autre(s) compartiment(s), le compartiment intermédiaire 4, un ou des éventuel(s) autre(s) compartiment(s) et le dernier compartiment 5 jusqu'à la sortie 6,
- le premier compartiment 3 comprend en outre une entrée 8 appropriée pour introduire un gaz, une sortie 9 appropriée pour évacuer un gaz et deux entrées distinctes 10 et 27 appropriées pour introduire un liquide,
- le compartiment intermédiaire 4 comprend une sortie 11 appropriée pour évacuer un gaz et une entrée 12 appropriée pour introduire un liquide,
- le dernier compartiment 5 comprend en outre une entrée 13 appropriée pour réguler le pH, une entrée 14 appropriée pour introduire un gaz et une entrée 15 appropriée pour introduire un liquide.

### Procédé de production d'azote minéral et/ou de dihydrogène à partir de diazote

La présente invention a également pour objet un procédé biologique de production d'azote minéral, en particulier de nitrates, à partir de l'azote atmosphérique, c'est-à-dire de diazote N2. Le procédé selon l'invention met en effet en oeuvre successivement des bactéries fixatrices d'azote et des bactéries du genre *Nitrosomonas* et/ou du genre *Nitrobacter.* De manière avantageuse, le procédé biologique de production d'azote minéral à partir de l'azote atmosphérique permet également la production de dihydrogène.

La présente invention a ainsi pour objet un procédé de production d'azote minéral, en particulier de nitrates et/ou de dihydrogène, à partir de diazote, caractérisé en ce qu'il comprend les étapes suivantes :
a) mise en incubation de matière organique, de diazote (N2), une source de sucre simple et de bactéries fixatrices d'azote dans un bioréacteur, pour obtenir un milieu de fermentation comprenant de l'ammoniac (NH3),
b) mise en incubation du milieu de fermentation obtenu à l'étape a) en présence d'eau et avec retrait de dihydrogène (H2) du bioréacteur, pour obtenir un milieu de fermentation comprenant de l'ammonium (NH₄⁺),
c) mise en incubation du milieu de fermentation obtenu à l'étape b) en présence de dioxygène (O₂) et de bactéries du genre *Nitrosomonas* et/ou du genre *Nitrobacter* dans le bioréacteur, pour obtenir un milieu de fermentation comprenant des nitrates (NO₃⁻),
d) optionnellement, séparation du milieu de fermentation obtenu à l'étape c) en une phase liquide et une phase solide et élimination de la phase solide, pour obtenir une solution riche en nitrates, et
e) optionnellement, concentration de la solution riche en nitrates obtenue à l'étape d), pour obtenir une solution concentrée en nitrates.

Le bioréacteur est, de préférence, un bioréacteur tel que défini ci-dessus dans la section « dispositif ».

### Etape a)

L'étape a) comprend la mise en incubation de matière organique, de diazote (N2), une source de sucre simple et de bactéries fixatrices d'azote dans un bioréacteur, pour obtenir un milieu de fermentation comprenant de l'ammoniac (NH3).

Lors de l'étape a), la matière organique, le diazote (N2), la source de sucre simple et/ou les bactéries fixatrices d'azote peuvent être introduits dans un bioréacteur préalablement à l'étape de mise en incubation, en continu ou de manière ponctuelle, par exemple à plusieurs instants séparés dans le temps.

La matière organique, la source de sucre simple, les bactéries et/ou le diazote peuvent être introduits dans le bioréacteur simultanément ou de manière séparée dans le temps.

La matière organique, la source de sucre simple, les bactéries fixatrices d'azote et/ou le diazote sont de préférence introduits par des entrées distinctes.

La matière organique est de préférence apportée sous la forme d'un liquide comprenant ladite matière organique et ayant une teneur en matière sèche comprise de 8% à 30%, de préférence de 10% à 18%, plus préférentiellement de 12% à 14%, par exemple 13%.

La matière organique peut être un digestat ou toute autre matière organique fermentescible.

Par « digestat », on désigne ici un sous-produit de la méthanisation, par exemple obtenu lors de la valorisation de déchets agricoles.

La matière organique telle que définie ci-dessus est de préférence une matière organique défibrée. Par « matière organique défibrée », on désigne ici une matière organique ayant par exemple subit au préalable une opération de broyage.

La matière organique telle que définie ci-dessus est de préférence une matière organique pasteurisée ou stérilisée.

La pasteurisation de la matière organique peut être effectuée pour toute méthode appropriée bien connue de l'homme du métier, par exemple à une température comprise de 62°C à 88°C pendant une durée déterminée, par exemple d'au moins 15 secondes.

La stérilisation de la matière organique peut être effectuée pour toute méthode appropriée bien connue de l'homme du métier, par exemple à une température supérieure à 100°C, par exemple 120°C, pendant une durée déterminée, par exemple d'au moins 20 minutes.

Par « sucre simple », on désigne ici un glucide de type monosaccharide (par exemple glucose, fructose, galactose ou mannose) ou disaccharide (par exemple saccharose, lactose ou maltose).

La source de sucre simple est par exemple sélectionnée dans le groupe consistant en une source de glucose, fructose, galactose, mannose, saccharose, lactose, maltose et leurs combinaisons.

La source de sucre simple est par exemple sélectionnée dans le groupe consistant en une solution comprenant ledit sucre simple, une mélasse de betterave, un déchet de pomme de terre et leurs combinaisons.

Une solution comprenant un sucre simple est de préférence une solution comprenant ledit sucre simple dissout dans de l'eau.

La source de sucre simple est de préférence une source de glucose, plus préférentiellement une solution de glucose.

L'étape a) est mise en oeuvre dans des conditions permettant la nitrogénase.

La nitrogénase est une réaction permettant la production d'ammoniac à partir de diazote.

L'étape a) est réalisée en conditions anaérobie, grâce à l'introduction du diazote sous pression dans le bioréacteur. Le diazote N2 introduit sous pression dans le bioréacteur permet en effet de chasser l'air résiduel contenu dans la matière organique. Le dioxygène est ainsi évacué du bioréacteur.

Le diazote est de préférence introduit dans le bioréacteur par injection sous pression.

Lors de l'étape a), la température dans le bioréacteur est de préférence maintenue à une température comprise de 30°C à 36°C, de préférence de 32°C à 34°C, par exemple 33°C.

Lors de l'étape a), la pression dans le bioréacteur est de préférence maintenue à une pression comprise de 0,1 ntm à 5 ntm, de préférence de 0,5 ntm à 2 ntm, par exemple 1,0 ntm de diazote.

Dans un mode de réalisation avantageux, lors de l'étape a), la température dans le bioréacteur est maintenue à 33°C et la pression à 1,0 ntm de diazote.

Par « ntm » ou «atm », on désigne ici une atmosphère normale. Une pression de 1 ntm correspond ainsi à 101325 Pa.

Lors de l'étape a), le pH dans le bioréacteur est de préférence libre, c'est-à-dire qu'il n'est pas ajusté à une valeur donnée ou dans une plage de valeurs donnée.

Par « bactéries fixatrices d'azote », on désigne ici des bactéries capables de capter l'azote atmosphérique et de le transformer en ammonium.

Les bactéries fixatrices d'azote sont de préférence sélectionnées dans le groupe consistant en des bactéries du genre *Azotobacter, Nitrobacter, Azospirillum, Acetobacter, Diazotrophicus, Clostridium, Klebsiella, Bacillus, Pseudomonas, Rhodobacter, Rhodospirillum, Synechoccus* et leurs combinaisons.

Des bactéries d'un ou plusieurs genres différents peuvent être utilisées dans l'étape a), par exemple des bactéries d'un seul genre, des bactéries d'au moins deux ou d'au moins trois genres différents.

Des bactéries d'une ou plusieurs espèces différentes d'un même genre peuvent être utilisées dans l'étape a), par exemple des bactéries d'une seule espèce, des bactéries d'au moins deux ou d'au moins trois espèces différentes d'un même genre.

### Etape b)

L'étape b) comprend la mise en incubation du milieu de fermentation obtenu à l'étape a) en présence d'eau et avec retrait de dihydrogène (H2) du bioréacteur, pour obtenir un milieu de fermentation comprenant de l'ammonium (NH₄⁺).

L'étape b) permet ainsi la dissolution de l'ammoniac (NH3) en ammonium (NH₄⁺). Cette réaction produit du dihydrogène qui est alors retiré du fermenteur.

Le procédé tel que défini ci-dessus permet ainsi la production de dihydrogène qui est récupéré lors de l'étape b), en particulier en le retirant du milieu de fermentation par une sortie appropriée pour évacuer un gaz.

L'étape b) est ainsi réalisée en conditions anaérobies.

Lors de l'étape b), la température dans le fermenteur est de préférence maintenue à une température comprise de 30°C à 36°C, de préférence de 32°C à 34°C, par exemple 33°C.

Lors de l'étape b), le pH dans le fermenteur est de préférence libre, c'est-à-dire qu'il n'est pas ajusté à une valeur donnée ou dans une plage de valeurs donnée.

Lors de l'étape b), la pression est de préférence laissée libre, c'est-à-dire qu'elle n'est pas ajustée à une valeur donnée ou dans une plage de valeurs donnée.

Dans un mode de réalisation avantageux, lors de l'étape b), la température est maintenue à 33°C et le pH libre dans le fermenteur.

### Etape c)

L'étape c) comprend la mise en incubation du milieu de fermentation obtenu à l'étape b) en présence de dioxygène (O₂) et de bactéries du genre *Nitrosomonas* et/ou du genre *Nitrobacter* dans le bioréacteur, pour obtenir un milieu de fermentation comprenant des nitrates (NO₃⁻).

L'étape c) permet la nitrification, c'est-à-dire la production de nitrates. La nitrification est réalisée par les bactéries du genre *Nitrosomonas* et/ou du genre *Nitrobacter qui* transforment notamment l'ammonium en nitrates.

Lors de l'étape c), la température dans le fermenteur est de préférence maintenue à une température comprise de 30°C à 36°C, de préférence de 32°C à 34°C, par exemple 33°C.

Lors de l'étape c), le pH est de préférence maintenu à un pH compris de 7 à 8, de préférence de 7,3 à 7,8, par exemple à pH 7,5.

Le pH est maintenu à la valeur souhaitée, de préférence par ajout de chaux (CaO), par exemple sous forme de lait de chaux.

Lors de l'étape c), la pression est de préférence laissée libre, c'est-à-dire qu'elle n'est pas ajustée à une valeur donnée ou dans une plage de valeurs donnée.

La concentration en nitrates est de préférence mesurée au cours de l'étape c).

### Etapes a), b) et c)

Dans un mode de réalisation avantageux de l'invention, les étapes a), b) et c) sont effectuées successivement dans un seul et même compartiment, tel que dans un bioréacteur comprenant une seule cuve de fermentation, ladite cuve de fermentation correspondant à un unique compartiment.

Dans un autre mode de réalisation avantageux de l'invention, les étapes a), b) et c) sont réalisées dans des compartiments séparés, par exemple respectivement dans un premier compartiment, un compartiment intermédiaire et un dernier compartiment, lorsque le procédé est mis en oeuvre au moyen d'un dispositif tel que défini ci-dessus dans la section du même nom.

Dans un mode de réalisation préféré, le bioréacteur comprend ainsi une cuve de fermentation comprenant (i) au moins trois compartiments successifs tels que définis ci-dessus ou (ii) au moins trois cuves de fermentation, chaque cuve de fermentation correspondant à un des compartiments successifs tels que définis ci-dessus.

Dans un autre mode de réalisation préféré, le procédé est mis en oeuvre dans trois bioréacteurs distincts, de préférence montés en série, chaque bioréacteur correspondant à un des compartiments successifs tels que définis ci-dessus.

L'étape a) est de préférence réalisée dans le premier compartiment 3, 3', 3" d'un dispositif tel que défini ci-dessus et, éventuellement, dans au moins un doublon de ce premier compartiment.

La matière organique, le diazote, la source de sucre simple et les bactéries fixatrices d'azote sont alors, de préférence, introduits dans le premier compartiment, par exemple en continu.

La vitesse d'introduction de la matière organique, du diazote, de la source de sucre simple et/ou des bactéries fixatrices d'azote peut varier au cours du procédé. La vitesse d'introduction est de préférence modulée en fonction de la concentration en ammonium mesurée dans le compartiment intermédiaire 4, 4', 4".

L'étape b) est de préférence réalisée dans le compartiment intermédiaire 4, 4', 4" d'un dispositif tel que défini ci-dessus et, éventuellement, dans au moins un doublon de ce compartiment intermédiaire.

L'introduction du milieu de fermentation comprenant de l'ammoniac (NH3) du premier compartiment vers le compartiment intermédiaire dépend de préférence de l'introduction de la matière (c'est-à-dire matière organique, diazote, source de sucre simple et bactéries fixatrices d'azote) dans le premier compartiment. Le milieu de fermentation se déplace ainsi du premier compartiment au compartiment intermédiaire, en étant « poussé » par la nouvelle matière entrante.

L'étape c) est, de préférence, réalisée dans le dernier compartiment 5, 5', 5" d'un dispositif tel que défini ci-dessus et, éventuellement, dans au moins un doublon de ce dernier compartiment.

L'introduction du milieu de fermentation comprenant de l'ammonium du compartiment intermédiaire vers le dernier compartiment dépend de préférence de l'introduction de la matière (c'est-à-dire matière organique, diazote, source de sucre simple et bactéries fixatrices d'azote) dans le premier compartiment. Le milieu de fermentation se déplace ainsi du premier compartiment au compartiment intermédiaire, puis au dernier compartiment en étant poussé par la nouvelle matière entrante dans le premier compartiment.

De manière similaire, la sortie du milieu de fermentation comprenant des nitrates du dernier compartiment par la sortie 6 dépend de préférence de l'introduction de la matière (c'est-à-dire matière organique, diazote, source de sucre simple et bactéries fixatrices d'azote) dans le premier compartiment. Le milieu de fermentation se déplace ainsi du premier compartiment au compartiment intermédiaire, puis au dernier compartiment jusqu'à la sortie 6, en étant poussé par la nouvelle matière entrante dans le premier compartiment.

### Etape d)

L'étape optionnelle d) comprend la séparation du milieu de fermentation comprenant des nitrates en une phase solide et une phase liquide, et l'élimination de la phase solide, pour obtenir une solution riche en nitrates.

L'étape d) est de préférence réalisée dans un dispositif permettant de séparer une phase solide d'une phase liquide, tel qu'une centrifugeuse ou un séparateur de phase.

La phase liquide correspond à une solution riche en nitrates.

La phase solide comprenant notamment les débris cellulaires peut être valorisée, par exemple par épandage directement aux champs.

### Etape e)

L'étape optionnelle e) comprend la concentration de la solution riche en nitrates, afin d'obtenir une solution concentrée en nitrates.

L'étape e) est de préférence réalisée dans un dispositif permettant de concentrer une solution, par exemple par filtration et/ou par osmose inverse.

Les a) à c) et les étapes optionnelles d) et e) sont avantageusement mises en oeuvre au moyen d'un dispositif tel que défini ci-dessus dans la section « dispositif».

### Utilisation du dispositif selon l'invention pour la production de nitrates et/ou de dihydrogène à partir de diazote

La présente invention a également pour objet l'utilisation d'un dispositif tel que défini ci-dessus dans la section « dispositif» pour la production d'azote minéral, en particulier sous la forme de nitrate NO₃⁻ et/ou pour la production de dihydrogène.

La présente invention a ainsi pour objet l'utilisation telle que définie ci-dessus pour la production biologique d'azote minéral, en particulier sous la forme de nitrate NO₃⁻, et/ou pour la production de dihydrogène à partir de diazote.

La présente invention a particulièrement pour objet l'utilisation telle que définie ci-dessus pour la production biologique d'azote minéral, en particulier sous la forme de nitrate NO₃⁻ et/ou de dihydrogène par (i) fermentation avec des bactéries fixatrices d'azote et/ou (ii) fermentation avec des bactéries du genre *Nitrosomonas* et/ou du genre *Nitrobacter.*

La présente invention a particulièrement pour objet l'utilisation telle que définie ci-dessus pour la production biologique d'azote minéral, en particulier sous la forme de nitrate NO₃⁻, par (i) fermentation avec des bactéries fixatrices d'azote et (ii) fermentation avec des bactéries du genre *Nitrosomonas* et/ou du genre *Nitrobacter.*

La présente invention a particulièrement pour objet l'utilisation telle que définie ci-dessus pour la production biologique de dihydrogène par a) fermentation avec des bactéries fixatrices d'azote permettant d'obtenir de l'ammoniac et b) dissolution de l'ammoniac en ammonium en présence d'eau, en particuler par ajout d'eau, permettant de produire du dihydrogène.

### Utilisation de bactéries pour la production d'azote minéral et/ou de dihvdroaène

L'invention a également pour objet l'utilisation de bactéries pour la production d'azote minéral, en particulier sous la forme de nitrate NO₃⁻, et/ou de dihydrogène à partir de diazote.

L'invention a particulièrement pour objet l'utilisation telle que définie ci-dessus, dans laquelle les bactéries sont des bactéries fixatrices d'azote et/ou des bactéries du genre *Nitrosomonas* et/ou du genre *Nitrobacter.*

L'invention a particulièrement pour objet l'utilisation telle que définie ci-dessus, dans laquelle (i) le diazote est transformé en ammoniac par des bactéries fixatrices d'azote, (ii) l'ammoniac est transformé en ammonium avec production de dihydrogène en présence d'eau et/ou (iii) l'ammonium est transformé en nitrates des bactéries du genre *Nitrosomonas* et/ou du genre *Nitrobacter.*

L'invention a particulièrement pour objet l'utilisation de de bactéries pour la production d'azote minéral, en particulier sous la forme de nitrate NO₃⁻, et/ou de dihydrogène à partir de diazote au moyen d'un dispositif tel que défini ci-dessus dans la section « dispositif ».

D'autres caractéristiques et avantages de l'invention ressortiront mieux des figures et de l'exemple qui suivent, donnés à titre illustratif et non limitatif.

### Figures

Les figures 1 à 3 sont des exemples schématiques d'un dispositif approprié pour la production de nitrates à partie de diazote.

Le dispositif présenté à la figure 1 comprend un bioréacteur 1, un dispositif 18 permettant de séparer une phase liquide d'une phase solide et un dispositif 21 permettant de concentrer une solution.

Les compartiments peuvent avoir une forme telle que présentée à la figure 1 ou une forme différente, par exemple sans les parties hachurées.

Le bioréacteur 1 comprend une cuve de fermentation 24 comprenant une entrée 2, par exemple pour introduire un liquide comprenant de la matière organique, et une sortie 6, par exemple pour évacuer un milieu de fermentation comprenant des nitrates, munies chacune d'un clapet anti-retour 7, de sorte qu'un liquide introduit dans la cuve par l'entrée 2 ne peut pas ressortir par cette entrée, et qu'un liquide sortant de la cuve par la sortie 6 ne peut pas retourner dans la cuve 24 par cette sortie. La cuve de fermentation 24 comprend au moins trois compartiments successifs 3, 4 et 5, le premier compartiment 3 étant relié à l'entrée 2 et le dernier compartiment 5 étant relié à la sortie 6. Chaque compartiment est relié au compartiment suivant, par au moins un clapet anti-retour 7, de sorte qu'un liquide introduit dans un compartiment successif ne peut retourner dans le compartiment précédent.

Un liquide introduit dans le premier compartiment 3 par l'entrée 2 passera donc successivement dans le ou les éventuels compartiments additionnels si la cuve de fermentation comprend plus de 3 compartiments, dans le compartiment intermédiaire 4, dans le ou les éventuels compartiments suivants si la cuve de fermentation comprend plus de 3 compartiments, puis dans le ou les éventuels compartiments suivants si la cuve de fermentation comprend plus de 3 compartiments dans le dernier compartiment 5, et enfin sortira de la cuve de fermentation par la sortie 6.

Le compartiment 3 comprend également une entrée 8 appropriée pour introduire un gaz, par exemple du diazote, une sortie 9 appropriée pour évacuer un gaz, par exemple du dioxygène, une entrée 10 appropriée pour introduire un liquide, par exemple comprenant des bactéries fixatrices d'azote et une entrée 27 appropriée pour introduire un liquide, par exemple une source de sucre simple. L'entrée 8 et la sortie 9 se situent au niveau de la partie supérieure du compartiment. Les entrées 2, 10 et 27 se situent par exemple sur la partie opposée libre du compartiment, les entrées 10 et 27 étant situées en dessous de l'entrée 2.

Le compartiment intermédiaire 4 comprend au moins une sortie 11 appropriée pour évacuer un gaz, par exemple du dihydrogène, et au moins une entrée 12 appropriée pour introduire un liquide, par exemple de l'eau. La sortie 11 se situe au niveau de la partie supérieure du compartiment. L'entrée 12 se situe, par exemple, au niveau de la partie supérieure du compartiment.

Le compartiment 5 comprend au moins une entrée 13 appropriée pour réguler le pH, par exemple appropriée pour introduire de la chaux, au moins une entrée 14 appropriée pour introduire un gaz, par exemple de dioxygène, et au moins une entrée 15 appropriée pour introduire un liquide, par exemple un liquide comprenant des bactéries du genre *Nitrosomonas* et/ou du genre *Nitrobacter.* L'entrée 14 se situe au niveau de la partie supérieure du compartiment. L'entrée 13 se situe par exemple sur la partie supérieure du compartiment et l'entrée 15 sur une partie latérale du compartiment.

La cuve de fermentation 24 comprend avantageusement un piston 16 relié à l'entrée 2 permettant d'injecter dans l'entrée 2 un liquide arrivant par l'entrée 17, par exemple un liquide comprenant de la matière organique.

Dans un mode de réalisation avantageux, un dispositif 18 permettant de séparer une phase liquide d'une phase solide, par exemple une centrifugeuse ou un séparateur de phase, est relié au bioréacteur 1 par la sortie 6 et comprend une sortie 20 appropriée pour évacuer un liquide, par exemple une solution riche en nitrates, et une sortie 19 appropriée pour évacuer une phase solide.

Dans un mode de réalisation avantageux, un dispositif 21 permettant de concentrer une solution, par exemple par filtration ou osmose inverse est relié au dispositif 18 permettant de séparer une phase liquide d'une phase solide par la sortie 20. Le dispositif 21 comprend une sortie 22 permettant de récupérer le rétentat, par exemple une solution concentrée en nitrates et autres formes azotées, et une sortie 23 permettant d'évacuer le perméat.

Le dispositif illustré à la figure 2 est une variante de celui présenté à la figure 1. Au lieu de comprendre une seule cuve de fermentation divisée en 3 compartiments, le bioréacteur de la figure 2 comprend trois cuves de fermentation distinctes 3', 4' et 5', chaque cuve de fermentation correspondant ainsi à un compartiment selon l'invention. Le premier compartiment 3' et le compartiment intermédiaire 4' sont reliés entre eux par une sortie 25, de préférence munie d'au moins un clapet anti-retour 7 qui peut être placé à la sortie du premier compartiment 3', à l'entrée du compartiment intermédiaire 4', ou dans la sortie 25, c'est-à-dire entre la sortie du premier compartiment 3' et l'entrée du compartiment intermédiaire 4'. Le compartiment intermédiaire 4' et le dernier compartiment 5' sont reliés entre eux par une sortie 26, de préférence munie d'au moins un clapet anti-retour 7 qui peut être placé à la sortie du compartiment intermédiaire 4', à l'entrée du dernier compartiment 5', ou dans la sortie 26, c'est-à-dire entre la sortie du compartiment intermédiaire 4' et l'entrée du dernier compartiment 5'.

Le dispositif illustré à la figure 3 est une variante de celui présenté à la figure 1 ou 2. En particulier, le dispositif présenté à la figure 3 comprend trois bioréacteurs distincts 3", 4", 5" montés en série, chaque bioréacteur correspondant ainsi à un compartiment selon l'invention. Le premier compartiment 3" et le compartiment intermédiaire 4" sont reliés entre eux par une sortie 25, de préférence munie d'au moins un clapet anti-retour 7 qui peut être placé à la sortie du premier compartiment 3", à l'entrée du compartiment intermédiaire 4", ou dans la sortie 25, c'est-à-dire entre la sortie du premier compartiment 3" et l'entrée du compartiment intermédiaire 4". Le compartiment intermédiaire 4" et le dernier compartiment 5" sont reliés entre eux par une sortie 26, de préférence munie d'au moins un clapet anti-retour 7 qui peut être placé à la sortie du compartiment intermédiaire 4", à l'entrée du dernier compartiment 5", ou dans la sortie 26, c'est-à-dire entre la sortie du compartiment intermédiaire 4" et l'entrée du dernier compartiment 5".

### EXEMPLE

Des bactéries fixatrices d'azote, une source de sucre simple (par exemple une solution de glucose), du diazote (N2) et un liquide comprenant 15% de matière sèche de matière organique défibrée pasteurisée sont introduits en continu dans le premier compartiment 3 du bioréacteur présenté à la figure 1, respectivement par les entrées 10, 27, 8 et 2, et forment un milieu de fermentation. La vitesse d'introduction est modulée en fonction de la concentration en NH4 mesurée dans le compartiment intermédiaire 4. En fixant le diazote, les bactéries fixatrices d'azote produisent de l'ammoniac (NH3) par neutrogénase dans le milieu de fermentation présent dans le premier compartiment 3. Cette étape est effectuée en conditions anaérobie, grâce à l'introduction de diazote sous pression et au retrait de dioxygène par la sortie 9, à 33°C et sous une pression de 1,0 ntm de diazote.

Le milieu de fermentation comprenant de l'ammoniac obtenu dans le premier compartiment 3 est introduit en continu dans le compartiment intermédiaire 4 qui est maintenu à une température de 33°C. L'introduction dans le compartiment intermédiaire 4 est régentée par l'introduction de la matière *(bactéries fixatrices d'azote, source de sucre simple, diazote et un liquide comprenant la matière organique)* dans le premier compartiment 3. Le milieu de culture se déplace ainsi dans chaque compartiment, en étant « poussé » par la nouvelle matière entrante. Le pH du milieu de fermentation du compartiment intermédiaire 4 est laissé libre. L'introduction dans le compartiment intermédiaire 4 d'eau par l'entrée 12 permet ainsi de transformer l'ammoniac en ammonium (NH₄⁺). Le dihydrogène (H2) produit par cette réaction est retiré du compartiment 4 par la sortie 11.

Le milieu de fermentation comprenant de l'ammonium obtenu dans le compartiment intermédiaire 4 est introduit en continu dans le dernier compartiment 5 qui est maintenu à une température de 33°C et à un pH de 7,5, si besoin par ajout de chaux par l'entrée 13. Comme expliqué précédemment, l'introduction dans le dernier compartiment 5 est régentée par l'introduction de la matière dans le premier compartiment 3. Du dioxygène (O₂) et des bactéries du genre *Nitrosomonas* et/ou du genre *Nitrobacter* sont introduits dans le dernier compartiment 5 respectivement par les entrées 14 et 15. Les bactéries transforment ainsi l'ammonium en nitrates.

Le milieu de fermentation comprenant des nitrates obtenu dans le dernier compartiment 5 est introduit dans un séparateur de phase 18. La phase solide est récupérée pour épandage. La phase liquide riche en nitrates est concentrée.

## Revendications

1. Procédé biologique de production d'azote minéral et/ou de dihydrogène à partir de diazote, **caractérisé en ce qu'**il comprend les étapes suivantes :
a) mise en incubation de matière organique, de diazote (N2), d'une source de sucre simple et de bactéries fixatrices d'azote dans un bioréacteur, pour obtenir un milieu de fermentation comprenant de l'ammoniac (NH3),
b) mise en incubation du milieu de fermentation obtenu à l'étape a) en présence d'eau et avec retrait de dihydrogène (H2) du bioréacteur, pour obtenir un milieu de fermentation comprenant de l'ammonium (NH₄⁺),
c) mise en incubation du milieu de fermentation obtenu à l'étape b) en présence de dioxygène (O₂) et de bactéries du genre *Nitrosomonas* et/ou du genre *Nitrobacter* dans le bioréacteur, pour obtenir un milieu de fermentation comprenant des nitrates (NO₃⁻),
d) optionnellement, séparation du milieu de fermentation obtenu à l'étape c) en une phase liquide et une phase solide et élimination de la phase solide, pour obtenir une solution riche en nitrates,
e) optionnellement, concentration de la solution riche en nitrates obtenue à l'étape d), pour obtenir une solution concentrée en nitrates.

2. Procédé selon la revendication 1, **caractérisé en ce que** les bactéries fixatrices d'azote sont sélectionnées dans le groupe consistant en des bactéries du genre *Azotobacter, Nitrobacter, Azospirillum, Acetobacter, Diazotrophicus, Clostridium, Klebsiella, Bacillus, Pseudomonas, Rhodobacter, Rhodospirillum, Synechoccus* et leurs combinaisons.

3. Procédé selon la revendication 1 ou 2, **caractérisé en ce que** l'étape a) est effectuée en conditions anaérobies, à une température comprise de 30°C à 36°C, et/ou à une pression comprise de 0,1 atm à 5 atm.

4. Dispositif approprié pour la mise en oeuvre d'un procédé biologique de production d'azote minéral et/ou de dihydrogène à partir de diazote, **caractérisé en ce que** :
- le dispositif comprend au moins trois compartiments successifs, le premier compartiment (3, 3', 3") étant relié à une entrée (2) et le dernier compartiment (5, 5', 5") étant relié à une sortie (6), lesdits compartiments successifs étant reliés entre eux de sorte qu'un liquide introduit par l'entrée (2) passera successivement dans le premier compartiment (3, 3', 3"), un ou des éventuel(s) autre(s) compartiment(s), le compartiment intermédiaire (4, 4', 4"), un ou des éventuel(s) autre(s) compartiment(s) et le dernier compartiment (5, 5', 5") jusqu'à la sortie (6),
- le premier compartiment (3, 3', 3") comprend en outre une entrée appropriée pour introduire un gaz (8), une sortie appropriée pour évacuer un gaz (9) et deux entrées appropriées pour introduire un liquide (10, 27),
- le compartiment intermédiaire (4, 4', 4") comprend une sortie appropriée pour évacuer un gaz (11) et une entrée appropriée pour introduire un liquide (12), et
- le dernier compartiment (5, 5', 5") comprend une entrée appropriée pour réguler le pH (13), une entrée appropriée pour introduire un gaz (14) et une entrée appropriée pour introduire un liquide (15).

5. Dispositif selon la revendication 4, **caractérisé en ce que** l'ensemble des compartiments successifs constitue une cuve de fermentation (24) ou **en ce que** chacun des compartiments successifs constitue une cuve de fermentation ou un bioréacteur.

6. Dispositif selon la revendication 4 ou 5, **caractérisé en ce qu'**il comprend en outre un dispositif (18) permettant de séparer une phase liquide d'une phase solide et/ou un dispositif (21) permettant de concentrer une solution.

7. Dispositif selon l'une des revendications 4 à 6, **caractérisé en ce qu'**il comprend un bioréacteur (1) et **en ce que** :
- ledit bioréacteur comprend une cuve de fermentation (24) comprenant une entrée (2) appropriée pour introduire un liquide et une sortie (6) appropriée pour évacuer un liquide,
- ladite cuve de fermentation comprend au moins trois compartiments successifs, le premier compartiment (3) étant relié à l'entrée (2) et le dernier compartiment (5) étant relié à la sortie (6), lesdits compartiments successifs étant reliés entre eux de sorte qu'un liquide introduit par l'entrée (2) passera successivement dans le premier compartiment (3), un ou des éventuel(s) autre(s) compartiment(s), le compartiment intermédiaire (4), un ou des éventuel(s) autre(s) compartiment(s) et le dernier compartiment (5) jusqu'à la sortie (6).

8. Procédé selon l'une des revendications 1 à 3, **caractérisé en ce qu'**il est mis en oeuvre dans un dispositif selon l'une des revendications 4 à 7.

9. Utilisation d'un dispositif selon l'une des revendications 4 à 7 pour la production d'azote minéral et/ou de dihydrogène à partir de diazote.

10. Utilisation de bactéries fixatrices d'azote et de bactéries du genre *Nitrosomonas* et/ou du genre *Nitrobacter* pour la production d'azote minéral et/ou de dihydrogène à partir de diazote dans un dispositif comprenant un ou plusieurs bioréacteurs.
